# EUROPÄISCHE PATENTSCHRIFT

(11) **EP 3 573 528 B1**
(45) Veröffentlichungstag und Bekanntmachung des Hinweises auf die Patenterteilung: **07.06.2023**
(21) Anmeldenummer: 17703685.2
(22) Anmeldetag: 30.01.2017
(51) Int. Cl.: A61B 5/08, A61B 5/145, A61B 5/1455, A61B 5/093, A61B 5/097

(54) **VORRICHTUNG ZUR BESTIMMUNG DER HÄMOGLOBINMENGE EINES PATIENTEN**
DEVICE FOR DETERMINING THE HEMOGLOBIN COUNT OF A PATIENT
DISPOSITIF SERVANT À DÉTERMINER LA QUANTITÉ D'HÉMOGLOBINE DANS LE SANG D'UN PATIENT

(43) Veröffentlichungstag der Anmeldung: 04.12.2019
(73) Patentinhaber: OPCO MEDICAL APS, 3460 Birkerød (DK)
(72) Erfinder: LUNDBY, Carsten, 3460 Birkerod (DK)
(74) Vertreter: Mader, Joachim
(86) Internationale Anmeldenummer: PCT/EP2017/051919
(87) Internationale Veröffentlichungsnummer: WO 2018/137780

(56) Entgegenhaltungen:
- WO-A1-03/100440
- CN-A- 103 263 715
- DE-A1-102006 057 185
- US-A1- 2009 071 481

## Beschreibung

### 1. Gebiet der Erfindung

Die vorliegende Erfindung betrifft eine Vorrichtung zur Bestimmung der Hämoglobinmenge eines Patienten.

### 2. Hintergrund der Erfindung

Hämoglobin ist der eisenhaltige Proteinkomplex der roten Blutkörperchen, der Sauerstoff binden und im Blutkreislauf transportieren kann. Aufgrund der zentralen Rolle in der Sauerstoff-Versorgung sind viele Krankheiten wie beispielsweise Anämie, Polyglobulie und Dehydration bzw. Hyperhydration assoziiert mit einer Änderung der Hämoglobinmenge im Blut. Für die Diagnose und Behandlung von Krankheiten kann die Hämoglobinmenge im Blut eines Patienten bestimmt werden. Dies kann über verschiedene invasive oder nicht-invasive Verfahren erfolgen.

Patent Dokument WO 03/100440 A1 offenbart eine Vorrichtung und ein Verfahren zur Bestimmung der Hämoglobinmenge mittels Inhalation einer vorgegebenen Menge an Kohlenmonoxid. Die Vorrichtung umfasst dabei ein Mundstück, welches über ein Verbindungsstück mit einem mit Sauerstoff befüllbaren Beutel verbunden ist.

Patent Dokument US 2015/0140670 A1 offenbart ein Verfahren und ein Diagnose-Kit, das eingerichtet ist um schnell und nicht-invasiv physiologische Hämoglobinspiegel zu bestimmen. Ein Diagnose-Kit kann dabei eine Kammer beinhalten, die mit einem Indikator vorgefüllt ist, wobei die Indikator-Lösung eine Tetramethylbenzidin (TMB) Lösung beinhaltet, wobei die Lösung eingerichtet ist um die Farbe zu wechseln. Weiter kann ein Aufnahmemittel vorgesehen sein um eine Probe eines Testsubjekts aufzunehmen.

Patent Dokument US 2009/0071481 A1 offenbart eine medizinische Vorrichtung zur Verabreichung von therapeutischem oder diagnostischem Gas. In einen Kohlenmonoxidreservoir mit einem bekannten Volumen wird der Druck gemessen um die Kohlenmonoxidmenge zu bestimmen. Ein Temperatursensor wird benutzt um die Druckmessung zu verbessern.

Im Stand der Technik kann der Hämoglobinwert im Blut eines Patienten mehr oder weniger genau bestimmt werden. Jedoch ist es wünschenswert den Hämoglobinwert möglichst genau zu bestimmen um die Diagnostik von Krankheiten und eine Behandlung der mit der Krankheit zusammenhängenden Symptome zu verbessern.

Es daher Aufgabe der vorliegenden Erfindung eine verbesserte Vorrichtung und ein Verfahren zur Bestimmung der Hämoglobinmenge eines Patienten bereitzustellen, welche zumindest teilweise die oben aufgeführten Probleme löst.

### 3. Ausführliche Beschreibung der Erfindung

Die oben aufgeführten Probleme werden durch eine Vorrichtung gemäß Anspruch 1 und ein Verfahren gemäß Anspruch 17 gelöst.

Die vorliegende Erfindung betrifft eine Vorrichtung zur Bestimmung der Hämoglobinmenge eines Patienten, aufweisend: ein geschlossenes Gas-Volumen; einen Gasauslass mit einem Mundstück, wobei das Mundstück eingerichtet ist, um ein Ein- und Ausatmen von Gas in und aus dem geschlossenen Gas-Volumen zu ermöglichen; ein Mittel zur Kohlenmonoxidzufuhr in das geschlossene Gas-Volumen; dadurch gekennzeichnet, dass das Mittel zur Kohlenmonoxidzufuhr ein Kohlenmonoxidreservoir aufweist, welches mit Sensoren zur Bestimmung von Temperatur und Druck des Kohlenmonoxids im Kohlenmonoxidreservoir versehen ist. Hämoglobin weist eine hohe Affinität für eine Bindung von Kohlenmonoxid (CO) auf. Die Bindungsaffinität des Hämoglobins ist etwa 200 bis 300 mal höher für Kohlenmonoxid als für Sauerstoff. Somit verdrängt eingeatmetes Kohlenmonoxid den Sauerstoff, der an das Hämoglobin im Blut eines Patienten gebunden ist. Durch eine Messung des an das Hämoglobin gebundenen Kohlenmonoxids kann auf den Gehalt bzw. die Menge von Hämoglobin im Blut eines Patienten geschlossen werden. Hierfür ist eine genaue Bestimmung der Menge des zugeführten Kohlenmonoxids notwendig. Die Kohlenmonoxid-Aufnahme des Patienten ist abhängig von der Hämoglobinmenge im Blut des Patienten und der verabreichten Menge an Kohlenmonoxid. Für die Bestimmung der Hämoglobinmenge ist es entscheidend zu wissen wie viel Kohlenmonoxid zum Einatmen bereitgestellt wurde, da eine höhere oder niedrigere Menge an bereitgestelltem und verabreichtem Kohlenmonoxid zu einer entsprechend höheren oder niedrigeren Menge an gebundenem Kohlenmonoxid im Blut des Patienten führen kann. Dementsprechend steigt die Ungenauigkeit bei der Bestimmung der Hämoglobinmenge mit der Ungenauigkeit bei der Bestimmung der Menge des verabreichten Kohlenmonoxids.

Bisher wurde das zu verabreichende Kohlenmonoxid manuell in Volumina von Spritzen aufgezogen und visuell kontrolliert. Eine exakte Bestimmung des so aufgezogenen Kohlenmonoxidvolumens und somit der in dem Volumen beinhalteten Kohlenmonoxidmenge ist hierbei nur grob möglich. Um den Fehler beim Aufziehen des Kohlenmonoxidvolumens zu reduzieren werdend die eingesetzten Spritzen durch wiederholtes Aufziehen des Kohlenmonoxidgases "gespült", damit kein Restgas, wie beispielsweise Umgebungsluft, mehr im Volumen der Spritze vorhanden ist. Dieser Vorgang kostet jedoch Zeit und es wird giftiges Kohlenmonoxid an die Umgebungsluft abgegeben. Daher muss hierbei unter einem Abzug gearbeitet werden, was bei einer Messung mit der erfindungsgemäßen Vorrichtung nicht notwendig ist, da das zugeführte Kohlenmonoxid das geschlossene Gas-Volumen nicht unkontrolliert verlassen kann. Zudem wird bei der herkömmlichen Methode unerwünschter Weise Kohlenmonoxid verbraucht, welches nicht für die Messung genutzt werden kann. Bei der vorliegenden Erfindung hingegen wird die Menge an Kohlenmonoxid, die in dem Kohlenmonoxidreservoir enthalten ist und dem geschlossenen Gas-Volumen zugeführt werden kann, exakt bestimmt. Im Gegensatz zur herkömmlichen Vorgehensweise kann die Kohlenmonoxidmenge durch das Messen des Drucks und der Temperatur genau bestimmt werden. Hierbei kann das Kohlenmonoxidreservoir aus einem Metall, vorzugsweise aus Aluminium bestehen, was eine genaue Bestimmung des vom Reservoir bereitgestellten Volumens ermöglicht. Es können aber auch andere geeignete Materialien für das Kohlenmonoxidreservoir verwendet werden, wie beispielsweise Kunststoffe, wie z.B. Polyoxymethylen. Durch die genaue Bestimmung der Menge von zugegebenem Kohlenmonoxid wird somit die Genauigkeit der Hämoglobinbestimmung verbessert. Die Notwendigkeit für ein "spülen" der Kohlenmonoxidreservoirs wird eliminiert, da es sich um ein geschlossenes Gassystem handelt und somit kann kein anderes Gas als das zugeführte Kohlenmonoxid im Reservoir vorhanden sein sollte. Zudem ist somit eine Zunahme des Drucks im Reservoir direkt mit einer zugegebenen Menge an Kohlenmonoxid verbunden und entsprechend kann eine im Reservoir verbleibende Restmenge an Kohlenmonoxid exakt bestimmt werden.

Durch die Messung der Temperatur des Kohlenmonoxids stellt die Vorrichtung zudem erfindungsgemäß sicher, dass die verabreichte Menge an Kohlenmonoxid unabhängig von der Umgebungstemperatur, die auf die Gastemperatur und damit auf das Volumen des Gases Einfluss haben kann, exakt bestimmt werden kann. Dies reduziert die Fehleranfälligkeit der Messung durch die erfindungsgemäße Vorrichtung. Durch das geschlossene Gas-Volumen und das von der Umgebung abgetrennte Kohlenmonoxidreservoir wird verhindert, dass giftiges Kohlenmonoxid vor oder während der Kohlenmonoxid Aufnahme durch den Patienten in die Umgebung gelangt. Somit ist die Vorrichtung geeignet ohne besondere zusätzliche Vorsichtsmaßnahmen betrieben zu werden und kann beispielsweise in einer Privatpraxis oder in einer Klinik in normalen Behandlungsräumen eingesetzt werden. Durch die exakte Bestimmung der zugegebenen Menge an Kohlenmonoxid wird somit eine genaue Hämoglobinbestimmung ermöglicht. Die Hämoglobinbestimmung kann zudem einfach und genau auch zu unterschiedlichen Zeitpunkten durchgeführt werden um beispielsweise Hämoglobinmengen im Blut von Patienten bei Krankheitsverläufen auch über einen längeren Zeitraum hinweg vergleichbar messen zu können. Weiterhin können die Bauteile sterilisier- und wiederverwendbar ausgebildet sein, was Materialkosten spart und Abfall vermeidet. Hinter dem Mundstück kann ein antibakterieller und/oder antiviraler Filter angeordnet sein, der die ausgeatmete Luft des Patienten filtert, bevor sie in das geschlossene Gas-Volumen eintritt. Somit wird eine virale bzw. bakterielle Kontamination des geschlossenen Gas-Volumens verhindert.

Das Gas-Volumen kann auch einen Feuchtigkeitsfilter aufweisen, wobei der Feuchtigkeitsfilter eingerichtet ist die Feuchtigkeit aus dem geschlossenen Gas-Volumen zu filtern, was vorteilhaft ist, da die feuchte und warme Luft, die abgeatmet wird, andernfalls unerwünschter Weise zu einer Tröpfchenbildung, beispielsweise an den Innenwänden des Gas-Volumens, führen kann. Somit kann eine Beschädigung der Bauteile der Vorrichtung durch die Feuchtigkeit oder ein Ansiedeln von Keimen oder Schimmelpilzen, die ein feuchtes Milieu bevorzugen, vermieden werden. Beispielsweise kann der Feuchtigkeitsfilter in der Nähe des Mundstücks angeordnet sein, so dass der ausgeatmeten Luft die enthaltene Feuchtigkeit sofort entzogen wird, bevor sie in das geschlossene Gas-Volumen gelangt. Oder aber ein elektrisch angetriebenes Gebläse kann an das geschlossene Gas-Volumen angeschlossen werden. So kann das Gas-Volumen vor oder nach einer Messung innerhalb von wenigen Minuten getrocknet werden indem Luft in das Gas-Volumen geblasen und aus einem Gasauslass aus dem Gas-Volumen geleitet wird.

In einer bevorzugten Ausführungsform ist das Mittel zur Kohlenmonoxidzufuhr eingerichtet eine definierte Menge an Kohlenmonoxid dem geschlossenen Gas-Volumen zuzuführen. Das Zuführen der definierten Menge an Kohlenmonoxid in das geschlossene Gas-Volumen kann durch eine Bereitstellung des Kohlenmonoxids automatisch erfolgen, ohne zusätzliche Einflussnahme durch den Benutzer, was die Genauigkeit und die Reproduzierbarkeit der Messung erhöht. Oder die Zugabe kann manuell durch einen Benutzer ausgelöst werden, was den Messvorgang flexibel gestaltet, so dass für unterschiedliche Rahmenbedingungen, beispielsweise für unterschiedliche Patienten oder unter Berücksichtigung weiterer Messparameter, der Zeitpunkt des Zuführens des Kohlenmonoxidgases zu dem Gas-Volumen frei gewählt werden kann.

In einer bevorzugten Ausführungsform ist ein Kohlendioxidfilter in dem geschlossenen Gas-Volumen angeordnet, der eingerichtet ist Kohlendioxid aus dem geschlossenen Gas-Volumen herauszufiltern. Da das Gas-Volumen geschlossen ist, reichert sich das Gas-Volumen während des Ein- und Ausatmens eines Patienten mit Kohlendioxid (CO₂) an. Eine zu hohe Kohlendioxidkonzentration kann jedoch gesundheitsschädlich sein und somit ist vorzugsweise ein Kohlendioxidfilter in dem Gas-Volumen angeordnet, der das überschüssige Kohlendioxid während des Messvorgangs aus dem Gas-Volumen herausfiltern kann. Der Filter kann austauschbar vorgesehen werden, so dass er leicht ausgewechselt werden kann, wenn die Speicherkapazität des Filters für CO₂ erreicht ist. Das Herausfiltern von CO₂ ermöglicht es auch, dass das Gas-Volumen über einen längeren Zeitraum von einem Patienten ein- bzw. ausgeatmet werden kann ohne dass sich die CO₂ Konzentration im Gas-Volumen unvorteilhaft erhöht.

In einer bevorzugten Ausführungsform weist die Vorrichtung ein Mittel zur Sauerstoffzufuhr auf, wobei das Mittel eingerichtet ist Sauerstoff dem geschlossenen Gas-Volumen zuzuführen. Durch die Sauerstoffzufuhr kann ein Patient das Gas in dem Gas-Volumen über einen längeren Zeitraum ein- und ausatmen, ohne dass nach einer gewissen Zeit der Sauerstoffgehalt unvorteilhaft niedrig wird. Das Mittel kann hierbei reinen Sauerstoff dem Gas-Volumen zuführen oder auch ein Mischgas mit einer geringeren Sauerstoff-Konzentration. Vorzugsweise kann die Sauerstoffkonzentration in dem geschlossenen Gas-Volumen konstant gehalten werden. Hierfür kann das Mittel zur Sauerstoffzufuhr auch einen Sensor aufweisen, der die Sauerstoffkonzentration in dem Gas-Volumen bestimmen kann. Die Sauerstoffzufuhr kann hierbei entweder konstant oder aber auch stoßweise erfolgen, damit die Sauerstoffkonzentration beispielsweise einen definierten Grenzwert nicht unterschreitet. Das Mittel zur Sauerstoffzufuhr kann dabei auch einen oder mehrere Druckminderer umfassen, was ermöglicht, dass ein Sauerstoffreservoir mit hohem Druck, beispielsweise eine Sauerstoff-Gasflasche, an die Vorrichtung angeschlossen werden kann. Das Mittel zur Sauerstoffzufuhr kann dabei über ein oder mehrere Leitungen, die beliebig flexibel oder starr ausgebildet sein können, mit dem Gas-Volumen verbunden sein, wobei beispielsweise unterschiedliche Leitungen genutzt werden können um während des Betriebs der Vorrichtung dem Gas-Volumen Sauerstoff zuzuführen, beispielsweise bei einem "Spülen" des geschlossenen Gas-Volumens mit Sauerstoff oder bei der Verabreichung von Kohlenmonoxid. Auch kann das Mittel zur Sauerstoffzufuhr eingerichtet sein, Sauerstoff zunächst mit dem zugeführten Kohlenmonoxid zu vermischen um das Gasgemisch anschließend dem Gas-Volumen zuzuführen. Das gemeinsame Zuführen hat den Vorteil, dass die verschiedenen Gase gleichzeitig dem Gas-Volumen zugeführt werden können, was die Betriebszeit während einer Messung verkürzen kann.

In einer bevorzugten Ausführungsform umfasst das Mittel zur Sauerstoffzufuhr einen elastischen Ballon, wobei der elastische Ballon im Betrieb ein maximales Füllvolumen von 6 Liter, vorzugsweise 4,5 Liter und besonders bevorzugt 3 Liter aufweist. Der elastische Ballon kann hierbei eine hohe Elastizität aufweisen was ein flexibles Einstellen auf unterschiedliche Füllvolumina ermöglicht. Dies kann beispielsweise nötig werden, wenn unterschiedliche Patienten mit unterschiedlichen Lungenvolumen mit der Vorrichtung vermessen werden sollen. So kann der Ballon sowohl für geringe Füllvolumen geeignet sein, wie sie beispielsweise bei Kindern notwendig sind, als auch für größere Füllvolumen wie z.B. bei Erwachsenen. Weiterhin kann der Ballon auch austauschbar vorgesehen werden, so dass Ballons mit unterschiedlichen Volumina in der Vorrichtung vorgesehen werden können. Der Ballon ist hierbei vorzugsweise so eingerichtet, dass ein leichtes Ein- bzw. Ausatmen durch einen Patienten ermöglicht wird. Folglich dürfen die Rückstellkräfte, die das Gas aus dem aufgeblasenen Ballon nach dem Ausatmen des Patienten zurück in die Lunge treiben, nicht unvorteilhaft groß sein. Auch kann der Ballon austauschbar eingerichtet sein, so dass, je nach Bedarf, unterschiedliche Ballons verwendet werden können. Der Ballon ist vorzugsweise beim Einatmen des Patienten soweit wie möglich entleert um ein Verbleiben von Kohlenmonoxid in dem Ballon zu vermeiden. Der Ballon kann dabei so an der Vorrichtung vorgesehen sein, dass seine Außenhülle beim Betrieb keinen Kontakt mit einer begrenzenden Fläche wie beispielsweise dem Boden hat. Dies hat den Vorteil, dass eine Ausdehnung des Ballons nicht eingeschränkt wird. Weiterhin kann die Ausdehnung und Kontraktion des Ballons im Betrieb auch aktiv gesteuert werden. So kann das Ein- und Ausatmen eines Patienten aktiv unterstützt werden, was insbesondere bei Patienten von Vorteil sein kann, die Probleme haben selbstständig zu atmen. Weiter ist durch eine aktive Steuerung des Ballons eine definierte Atmung, beispielsweise über eine definierte Atemfrequenz oder Atemvolumen, möglich, so dass beispielsweise das über einen bestimmten Zeitraum ein- bzw. ausgeatmete Gas aus dem Gas-Volumen genau bestimmt werden kann, was die Reproduzierbarkeit von Messungen erhöht. Weiterhin kann der Ballon dazu verwendet werden das Lungenvolumen des Patienten zu bestimmen, indem das vom Patienten ausgeatmete Volumen, welches entsprechend das Volumen des Ballons vergrößert, bestimmt wird.

In einer bevorzugten Ausführungsform weist die Vorrichtung ein Mittel zur Bestimmung des Füllvolumens des elastischen Ballons auf, wobei das Mittel vorzugsweise ein optisches Mittel ist, das eingerichtet ist einen Abstand zwischen dem optischen Mittel und der Hülle des elastischen Ballons zu messen. Das Mittel zur Bestimmung des Füllvolumens kann eingesetzt werden um eine Detektion des Gas-Volumens während des Betriebs zu ermöglichen. Der Ballon kann dabei eine definierte Ausdehnung haben, abhängig von dem Füllvolumen. Weiter kann der Abstand zwischen des Außenhülle des Ballons und dem Mittel zur Bestimmung des Füllvolumens beispielsweise über eine optische Abstandsmessung erfolgen, wie eine Laufzeitmessung eines reflektierten Lichtpulses. Hierfür kann der Abstand zwischen der Ballonhülle und dem Mittel zur Bestimmung des Füllvolumens auf definierte Füllvolumina geeicht werden, so dass ein bestimmter Abstand jeweils einem bestimmten Füllvolumen entspricht. Die Steuerung für das Mittel zur Bestimmung des Füllvolumens kann dabei beispielsweise auch mit der Steuerung für das Mittel zur Sauerstoffzufuhr und/oder der Steuerung für die Mittel zur Kohlenmonoxidzufuhr verbunden sein um den Prozess des Ein- bzw. Ausatmens genau zu steuern. Bei einem aktiv betriebenen Ballon kann dieses ebenfalls mit dem Mittel zur Bestimmung des Füllvolumens des Ballons verbunden sein um das Füllvolumen während des Atemvorgangs zu steuern.

In einer bevorzugten Ausführungsform umfasst das optische Mittel zumindest einen Laser und einen Detektor, wobei der Laser und der Detektor unbeweglich an der Vorrichtung angeordnet sind. Die Außenhülle des Ballons ist dabei geeignet einen Lichtpuls, der von dem Laser ausgesandt wurde an einen Detektor zurück zu reflektieren. Laser und Detektor können dabei einen definierten Abstand zum Ballon aufweisen durch das unbewegliche Anbringen an der Vorrichtung. Entsprechend kann der Ballon unbeweglich an der Vorrichtung angeordnet sein, beispielsweise, indem er eine Aufhängung aufweist, die eine Ausdehnung bzw. Kontraktion des Ballons ermöglicht, aber die relative Position des Ballons zur Vorrichtung kontant hält. Somit wird bei einem ebenfalls unbeweglich angebrachten Laser und Detektor ein fehlerhaftes Bestimmen des Füllvolumens des Ballons vermieden, da die relativen Positionen von Ballon, Laser und Detektor zueinander konstant sind.

In einer bevorzugten Ausführungsform weist die Vorrichtung einen Kohlenmonoxiddetektor auf, der eingerichtet ist den Kohlenmonoxidgehalt des geschlossenen Gas-Volumens zu detektieren. Der Kohlenmonoxiddetektor kann dabei den Kohlenmonoxidgehalt messen, welcher beispielsweise nach Zugabe von Kohlenmonoxid und ein- und ausatmen des Gases durch den Patienten im Gas-Volumen verblieben ist. So kann sichergestellt werden, dass das zugeführte Kohlenmonoxid auch vom Patienten aufgenommen wurde. Weiterhin kann die bestimmte Menge des vom Patienten aufgenommenen Kohlenmonoxids durch die noch im Gas-Volumen verbliebene Menge von Kohlenmonoxid korrigiert werden, was den Fehler bei der Bestimmung des Hämoglobinwertes reduziert.

In einer bevorzugten Ausführungsform weist die Vorrichtung einen weiteren Gasauslass auf, wobei der Kohlenmonoxiddetektor an dem weiteren Gasauslass angeordnet ist. Der weitere Gasauslass kann ein Ausatmen durch den Patienten in die Umgebung ermöglichen. Dies kann zum Beispiel zum Ende einer Messung von Vorteil sein, wenn die Kohlenmonoxid-Restmenge, die nicht vom Patienten aufgenommen wurde, gemessen werden soll. Hierbei kann der elastische Ballon vollständig entleert sein, so dass sämtliches Restgas aus dem Gas-Volumen in die Umgebung austreten kann. Somit ist eine exakte Messung des im Gas-Volumen enthaltenen Kohlenmonoxids am weiteren Gasauslass möglich.

In einer bevorzugten Ausführungsform ist das Mittel zur Kohlenmonoxidzufuhr und/oder das Mittel zur Sauerstoffzufuhr und/oder die Gasauslässe über Ventile mit dem geschlossenen Gas-Volumen verbunden, wobei die Ventile separat regelbar sind. Eine separate Regelung der Ventile ermöglicht es eine jeweilige Zufuhr oder einen Auslass von Gas in bzw. aus dem Gas-Volumen zu steuern. Hierbei kann je nach Art des Vorgangs, beispielswiese beim Zuführen von Sauerstoff oder Kohlenmonoxid oder beim Entleeren des Gas-Volumens, unterschiedliche Ventile unterschiedlich lange oder zu unterschiedlichen Zeiten während des Betriebs der Vorrichtung geöffnet bzw. geschlossen werden. Dies ermöglicht eine flexible Anpassung im Betrieb, beispielsweise für Messungen mit unterschiedlichen Messparametern.

In einer bevorzugten Ausführungsform weist der zumindest eine Drucksensor des Kohlenmonoxidreservoirs eine Messungenauigkeit von <100 mbar, vorzugsweise <50 mbar und besonders bevorzugt <20 mbar auf. In einer bevorzugten Ausführungsform weist der zumindest eine Temperatursensor des Kohlenmonoxidreservoirs eine Messungenauigkeit von <2 °C, vorzugsweise <1,2 °C und besonders bevorzugt <0,6 °C auf. Der Druck und die Temperatur können hierbei elektronisch gemessen werden, womit die Bestimmung der Kohlenmonoxidmenge unabhängig von einem Benutzer erfolgen kann, was den Fehler bei der Bestimmung der Hämoglobinmenge im Blut eines Patienten weiter reduzieren kann.

In einer bevorzugten Ausführungsform weist die Vorrichtung eine Steuereinrichtung auf, wobei die Steuereinrichtung das Volumen des Kohlenmonoxids im Kohlenmonoxidreservoir bestimmt mit einer Messungenauigkeit von <10 ml, vorzugsweise <5 ml und besonders bevorzugt <2,4 ml. Die Bestimmung des Volumens des Kohlenmonoxids und/oder der Temperatur und/oder des Drucks kann hierbei über eine entsprechende Anzeige erfolgen, die von außerhalb der Vorrichtung ablesbar angeordnet sein kann. Die Vorrichtung kann auch eine entsprechende Schnittstelle für ein Senden der ermittelten Werte, beispielsweise an einen Computer aufweisen.

In einer bevorzugten Ausführungsform ist das Mundstück austauschbar. Somit kann die Vorrichtung schnell und hygienisch für unterschiedliche Patienten eingerichtet werden. Es können beispielsweise sterile Einwegmundstücke verwendet werden, die beispielsweise antibakterielle bzw. antivirale Filter umfassen können.

In einer bevorzugten Ausführungsform weist das Mittel zur Kohlenmonoxidzufuhr und/oder das Mittel zur Sauerstoffzufuhr zumindest einen regelbaren Anschluss auf zum Zuführen und/oder Abführen von Kohlenmonoxid bzw. Sauerstoff. Die Zufuhr von Kohlenmonoxid bzw. Sauerstoff kann hierbei über Gas-Speichermedien mit größeren Volumina bzw. Drücken, wie beispielsweise Gasflaschen, erfolgen. Somit kann die Vorrichtung eine lange Zeit betrieben werden, ohne dass die angeschlossenen Gas-Speichermedien ausgetauscht werden müssen. Die regelbaren Anschlüsse der Gas-Speichermedien können dabei Druckminderer aufweisen, die ein gefahrloses Befüllen der Mittel zur Kohlenmonoxidzufuhr und/oder Sauerstoffzufuhr ermöglichen. Zudem können die regelbaren Anschlüsse dazu dienen die Gas-Speichermedien zu verschließen, was ein unerwünschtes Austreten der Gase verhindern und so die Sicherheit im Betrieb oder der Lagerung erhöhen kann.

In einer bevorzugten Ausführungsform sind alle Bauteile der Vorrichtung außer dem Mundstück und dem Kohlendioxidfilter in einem Gehäuse angeordnet. Die vorteilhafte Anordnung in einem Gehäuse ermöglicht es die einzelnen Bestandteile der Vorrichtung vor unerwünschten äußeren Einflüssen wie Staub und Schmutz zu schützen. Vorteilhaft kann das Gehäuse aus einem starren und zugleich leichten Material wie beispielsweise Aluminium ausgebildet sein um vor Schäden die beherbergten Bauteile vor Schäden z.B. durch Schläge oder Stöße zu schützen. Weiter kann das Gehäuse so ausgebildet sein, dass die Vorrichtung für einen sterilen Betrieb geeignet ist. Hierfür kann es entsprechend vorteilhaft abgedichtet und ggf. an seiner Außenseite beschichtet sein. Weiterhin kann die Vorrichtung Sicherheitsmechanismen aufweisen, die ein unerwünschtes Austreten von Kohlenmonoxid verhindern können. So können die Ventile so geregelt sein, dass sie schließen, wenn ein gemessener Grenzwertwert einer im Gehäuse angeordneten Messvorrichtung für Kohlenmonoxid überschritten wird oder wenn der Druck in dem Kohlenmonoxidreservoir während des Befüllens zu langsam steigt, was auf ein Leck hindeuten kann oder wenn der Druck in dem Kohlenmonoxidreservoir einen Grenzwert übersteigt.

Die vorliegende Erfindung betrifft außerdem ein Verfahren zur Bestimmung einer Hämoglobinmenge unter Verwendung einer Vorrichtung gemäß einer der vorhergehenden Ausführungsformen, wobei das Verfahren die Schritte aufweist: i) Zuführen einer definierten Menge von Kohlenmonoxid zu dem geschlossenen Gas-Volumen; ii) Ein- und Ausatmen des Gases im geschlossenen Gas-Volumen durch den Patienten; iii) Bestimmen der Menge von Kohlenmonoxid im Blut des Patienten; iv) Bestimmen des Hämoglobin-Wertes anhand der Menge von Kohlenmonoxid im Blut des Patienten. Hierbei kann der Hämoglobinwert bestimmt werden indem dem Patienten zumindest eine Blutprobe vor der Verabreichung des Kohlenmonoxids entnommen wird und zumindest eine Blutprobe nach der Verabreichung des Kohlenmonoxids. Der exakte Zeitpunkt der Blutentnahme kann dabei variiert werden. Die Hämoglobinwert kann dann aus der Differenz des Kohlenmonoxidgehalts der jeweiligen Blutprobe bestimmt werden. Weiterhin kann der Kohlenmonoxidgehalt im Blut auch nicht-invasiv bestimmt werden, beispielsweise über optische Mittel.

In einer bevorzugten Ausführungsform wird die Hämoglobinmenge des Patienten mit einer Ungenauigkeit von <2,5%, vorzugsweise <1,5% und besonders bevorzugt <1% bestimmt. Eine solche Genauigkeit kann bei der Diagnose von Krankheiten vorteilhaft sein. Weiter kann eine solche Genauigkeit bei der Ausgestaltung einer Therapie vorteilhaft sein, die z.B. abhängig ist von dem Hämoglobinwert des Patienten und die somit genau auf den jeweiligen Patienten zugeschnitten werden kann. Eine solche Genauigkeit ermöglicht zudem auch langfristig angelegte Messungen, beispielsweise zur Beobachtung eines Pateinten über mehrere Jahre um so Krankheitsverläufe präziser darzustellen und unter anderem das An- oder Fehlschlagen einer Therapie zu bestimmen und diese ggf anzupassen.

In einer bevorzugten Ausführungsform weist das Verfahren vor dem Schritt i) die Schritte auf: Zuführen von reinem Sauerstoff in das geschlossene Gas-Volumen; und Ein- und Ausatmen des zugeführten reinen Sauerstoffs durch den Patienten. Dieses sogenannte "Spülen" mit reinem Sauerstoff hat den Vorteil, dass das Hämoglobin vor der Verabreichung von Kohlenmonoxid vorzugsweise den zugeführten reinen Sauerstoff in der Lunge bindet. Somit kann erreicht werden, dass so viel Sauerstoff wie möglich im Körper eines Patienten vorhanden ist, was das Ein- und Ausatmen des Patienten während der anschließenden Verabreichung von Kohlenmonoxid erleichtert. Auch wird der Grad einer Hypoxie reduziert, welche durch die Verabreichung von Kohlenmonoxid induziert werden kann. Somit kann das Kohlenmonoxid einem breiteren Spektrum von Patienten verabreicht werden.

In einer bevorzugten Ausführungsform erfolgt in dem Verfahren das Ein- und Ausatmen des Sauerstoffs vor dem Schritt i) für eine erste Zeit und das Ein- und Ausatmen des Gases in Schritt ii) für eine zweite Zeit, wobei die erste Zeit 0,1-6 Minuten beträgt, vorzugsweise 1-4 Minuten und wobei die zweite Zeit 1-240 Minuten beträgt, vorzugsweise 2-10 Minuten, besonders bevorzugt 6-10 Minuten. Je nach gewünschtem Messverfahren oder je nach Patient können die Messzeiten geeignet eingestellt werden. Vorzugsweise kann die erste Zeit ausreichend sein um den Patienten bzw. das Hämoglobin im Blut des Patienten entsprechend vorzubereiten, so dass das Hämoglobin, wie oben beschrieben, in einer geeignet definierten Art und Weise vorliegt und weiter kann die zweite Zeit ausreichend sein, dass nahezu das gesamte zugeführte Kohlenmonoxid vom Patienten durch das Ein- bzw. Ausatmen aufgenommen wurde.

### 4. Beschreibung der Figur

Zum besseren Verständnis der vorliegenden Erfindung und um die praktische Anwendbarkeit zu verdeutlichen wird im Folgenden eine Figur bereitgestellt und darauf Bezug genommen. Es sollte verstanden werden, dass die Figur nur eine beispielhafte Ausführungsform darstellt und somit in keiner Weise den Umfang der beanspruchten Erfindung einschränkt.

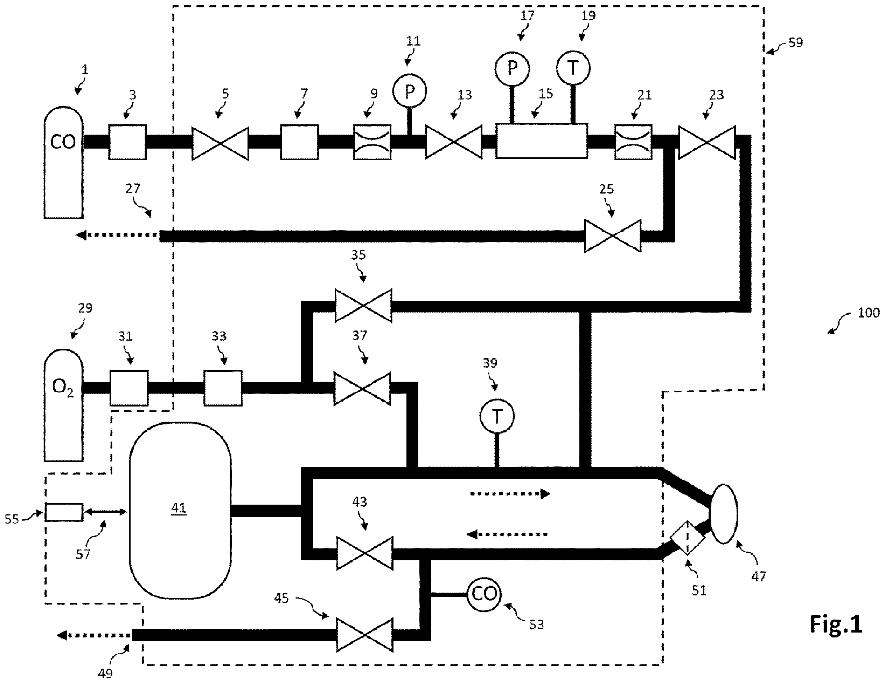

- Fig. 1: zeigt den schematischen Aufbau der Vorrichtung zur Bestimmung der Hämoglobinmenge.

### 5. Ausführliche Beschreibung der bevorzugten Ausführungsform

Im Folgenden wird die vorliegende Erfindung detailliert beschrieben unter Bezugnahme auf die beigefügte Figur, welche eine beispielhafte Ausführungsform der vorliegenden Erfindung darstellt. Es ist jedoch möglich, dass die vorliegende Erfindung auch in unterschiedlicher Art und Weise ausgestaltet ist, so dass die nachfolgend aufgeführte Ausführungsform nicht als einschränkend für den Schutzumfang der Erfindung zu betrachten ist. Vielmehr sollte die aufgeführte Ausführungsform einem Fachmann beispielhaft den Umfang der Erfindung verdeutlichen.

**Fig. 1** zeigt den schematischen Aufbau der Vorrichtung zur Bestimmung der Hämoglobinmenge bei einem Patienten. Hierbei ist eine CO-Quelle 1 über einen ersten Druckminderer 3 an ein CO-Sicherheitsventil 5 angeschlossen. Die CO-Quelle 1 kann beispielsweise eine CO-Gasflasche sein bei welcher eine große Menge CO-Gas mit einem hohen Druck komprimiert ist. Die Verbindung kann beispielsweise über einen gasdichten elastischen Schlauch erfolgen, der es ermöglicht, dass die CO-Quelle 1 auch weit entfernt von den restlichen Bauteilen der Vorrichtung angeordnet ist. So kann die CO-Quelle 1 beispielsweise in einem dafür geeigneten Lagerraum gelagert werden, während sich die restlichen Bauteile der Vorrichtung in einem normalen Behandlungszimmer befinden können. Das CO-Sicherheitsventil 5 ist wiederum an einen zweiten CO-Druckminderer 7 angeschlossen. Weiter ist der zweite CO-Druckminderer 7 über eine erste Drossel 9 und ein Ventil 13 mit dem CO-Reservoir 15 verbunden. Der Druck wird von einem Drucksensor 11 zwischen der ersten Drossel 9 und dem Ventil 13 detektiert. Durch die vorgeschalteten Ventile und Drosseln kann verhindert werden, dass das CO-Gas mit einem zu hohen Druck in das nachfolgende System eingespeist wird. Somit werden Schäden durch einen zu hohen Druck an den nachfolgenden Bauteilen des Systems vermieden. Auch kann ein ungewolltes Austreten des giftigen CO-Gases vermieden werden.

Der Drucksensor 11 stellt sicher, dass das CO-Reservoir 15 mit einem geeigneten Druck befüllt werden kann. Druck und Temperatur des CO in dem CO-Reservoir 15 werden über jeweilige Sensoren 17, 19 detektiert. Bei bekanntem Volumen des CO-Reservoirs 15 kann daraus die exakte Menge an zugeführtem CO bestimmt werden. Das CO-Reservoir 15 ist wiederum mit einer zweite Drossel 21 verbunden. Das CO-Reservoir 15 kann mit CO gespült werden indem das CO über ein Ventil 25 zu einem Auslass 27 geleitet wird. Somit kann das CO-Reservoir 15 vor jedem Betrieb der Vorrichtung in einen definierten Ausgangszustand gebracht werden. Die zweite Drossel 21 ist weiter über ein Ventil 23 mit dem geschlossenen Gas-Volumen 100 verbunden. Somit kann eine definierte Menge CO aus dem CO-Reservoir 15 in das geschlossene Gas-Volumen 100 geleitet werden. Weiter ist eine O₂-Quelle 29 über einen ersten O₂-Druckminderer 31 mit einem zweiten O₂-Druckminderer 33 verbunden. Die O₂-Quelle 29 kann beispielsweise eine O₂-Gasflasche sein, bei welcher eine große Menge Gas mit einem hohen Druck komprimiert ist. Wie auch die CO-Quelle 1 kann die O₂-Quelle 29 über einen gasdichten Schlauch verbunden werden, was die Anordnung der O₂-Quelle 29 flexibel gestaltet. Die Druckminderer 31, 33 verhindern, dass das O₂₋Gas mit einem zu großen Druck in das geschlossene Gas-Volumen 100 eingespeist wird. Während ein Patient in einem ersten Zeitraum reines O₂-Gas atmet (auch als "Spülung" mit O₂ bezeichnet) ist das mit dem zweiten Druckminderer 33 verbundene Ventil 35 geöffnet und O₂ wird dem geschlossenen Gas-Volumen 100 zugeführt. Anschließend kann CO in das geschlossene Gas-Volumen zugeführt werden und das Ventil 35 kurz geöffnet werden um durch das einströmende O₂ das in den zuführenden Verbindungen eventuell verbliebene CO in das geschlossene Gas-Volumen zu spülen. Während ein Patient das mit CO angereicherte Gas in einem bestimmten zweiten Zeitraum atmet ist das Ventil 35 geschlossen und O₂ wird dem geschlossenen Gas-Volumen 100 über das Ventil 37 zugeführt, bis eine gewünschte Sauerstoffkonzentration in dem Gas des geschlossenen Gas-Volumens 100 vorliegt.

Die Temperatur des Gases im geschlossenen Gas-Volumen 100 wird über einen Temperatursensor 39 gemessen. Ein Patient atmet das Gas im geschlossen Gas-Volumen 100 über das Mundstück 47 ein und aus, in Richtung der Pfeile im geschlossen Gas-Volumen 100. Das ausgeatmete Gas wird über einen im geschlossenen Gas-Volumen angeordneten CO₂-Filter 51 gefiltert. Somit wird ein Anreichern von CO₂-Gas im geschlossenen Gas-Volumen 100 vermieden. Während des Ein- und Ausatmens, nach Zugabe des COs, ist ein erstes Ventil 43 geöffnet, welches ein Ein- und Ausatmen in einen angeschlossenen elastischen Ballon 41 ermöglicht. Ein zweites Ventil 45 des geschlossenen Gas-Volumens 100, welches das geschlossene Gas-Volumen 100 mit einem Auslass 49 verbindet ist dabei geschlossen. Der elastische Ballon 41 dehnt sich bei einem Ausatmen des Patienten aus und zieht sich bei einem Einatmen des Patienten zusammen. Über den Abstand 57 zu der Außenhülle des elastischen Ballons 41, der von einem optischen Abstandsdetektor 55 bestimmt wird, kann ermittelt werden wie viel Gas-Volumen sich in dem elastischen Ballon 41 befindet. Durch die Differenz der Volumina beim Ein- und Ausatmen kann auch das Lungenvolumen des Patienten bestimmt werden. Weiterhin kann über die zeitliche Volumenänderung des elastischen Ballons 41 die Atemfrequenz des Patienten bestimmt werden. Nachdem der Patient das mit CO angereicherte Gas des geschlossenen Gas-Volumens 100 eine bestimmte Zeit ein- und ausgeatmet hat wird das erste Ventil 43 des geschlossenen Gas-Volumens 100 geschlossen und das zweite Ventil 45 wird geöffnet. Somit wird das Gas aus dem geschlossenen Gassystem 100 vom Patienten aus dem Auslass 49 ausgeatmet.

Vor dem zweiten Ventil 45 ist ein CO-Detektor 53 angeordnet, der die CO-Menge des abgeatmeten Gases detektieren kann. Durch die Messung des COs des abgeatmeten Gases kann bestimmt werden wie viel CO vom Patienten nicht aufgenommen wurde. Dieser Wert kann zur Korrektur des Wertes der dem Patienten zugeführten CO-Menge genutzt werden. Die Bauteile der Vorrichtung können in einem Gehäuse 59 angeordnet sein und die CO- und O₂-Quellen und die ersten Druckminderer 3, 31 können außerhalb des Gehäuses 59 gelagert sein und über entsprechende Anschlüsse mit den Bauteilen im Gehäuse 59 angeschlossen sein. Dies ermöglicht eine separate Lagerung und somit einen einfachen Austausch der CO- bzw. O₂-Quellen. Die nicht benötigten Abgase können über entsprechende Auslässe 27, 49 aus dem Gehäuse 59 geleitet werden. Weiter ist der elastische Ballon 41 so im Gehäuse 59 angeordnet, dass sein Abstand zu einem fest am Gehäuse 59 angeordneten optischen Abstandsdetektor 55 detektiert werden kann, was eine Bestimmung des Füllvolumens des elastischen Ballons 41 ermöglicht. Zur Bestimmung der Hämoglobinmenge wird dem Patienten vor dem Verabreichen von CO eine Blutprobe abgenommen und die Konzentration des an das Hämoglobin gebundenen COs bestimmt. Nachdem dem Patienten das CO verabreicht wurde und er das mit CO angereicherte Gas eine bestimmte Zeit geatmet hat wird ihm erneut eine Blutprobe abgenommen und die Konzentration des an das Hämoglobin gebundenen COs bestimmt. Die Konzentration des an das Hämoglobin gebundenem COs kann auch nicht-invasiv bestimmt werden, beispielsweise über optische Methoden. Aus den beiden Messungen des an das Hämoglobin gebundenen COs kann anschließend die Hämoglobinmenge im Blut des Patienten bestimmt werden.

**Bezugszeichen**

| Bezugszeichen | Teil |
|---|---|
| 1 | CO-Quelle |
| 3 | CO-Druckminderer 1 |
| 5 | CO-Sicherheitsventil |
| 7 | CO-Druckminderer 2 |
| 9 | Drossel 1 |
| 11 | Drucksensor vor Reservoir |
| 13 | Ventil vor Reservoir |
| 15 | CO-Reservoir |
| 17 | CO-Reservoir Drucksensor |
| 19 | CO-Reservoir Temperatursensor |
| 21 | Drossel 2 |
| 23 | Ventil f. CO-Dosierung |
| 25 | Ventil f. CO-Spülung |
| 27 | Auslass zum Spülen d. Reservoirs mit CO |
| 29 | O₂-Quelle |
| 31 | O₂-Druckminderer 1 |
| 33 | O₂-Druckminderer 2 |
| 35 | Ventil f. O₂-Spülung |
| 37 | Ventil f. O₂-Dosierung |
| 39 | Temperatursensor d. Gas-Volumens |
| 41 | elastischer Ballon |
| 43 | Ventil 1 Gas-Volumen |
| 45 | Ventil 2 Gas-Volumen |
| 47 | Mundstück |
| 49 | Auslass d. Gas-Volumens |
| 51 | CO₂-Filter |
| 53 | CO-Detektor |
| 55 | optischer Abstandsdetektor |
| 57 | Abstand zu Ballon |
| 59 | Gehäuse |
| 100 | geschlossenes Gas-Volumen |

## Patentansprüche

1. Eine Vorrichtung zur Verabreichung von Kohlenmonoxid für eine Bestimmung der Hämoglobinmenge eines Patienten, aufweisend:
ein geschlossenes Gas-Volumen (100);
einen Gasauslass mit einem Mundstück (47), wobei das Mundstück (47) eingerichtet ist um ein Ein- und Ausatmen von Gas in und aus dem geschlossenen Gas-Volumen (100) zu ermöglichen;
ein Mittel zur Kohlenmonoxidzufuhr in das geschlossene Gas-Volumen (100);
wobei das Mittel zur Kohlenmonoxidzufuhr ein Kohlenmonoxidreservoir (15) mit einem Volumen aufweist, welches mit Sensoren zur Bestimmung von Temperatur (19) und Druck (17) des Kohlenmonoxids im Kohlenmonoxidreservoir (15) versehen ist, wobei das Mittel zur Kohlenmonoxidzufuhr eingerichtet ist eine definierte Menge an Kohlenmonoxid dem geschlossenen Gas-Volumen (100) zuzuführen,
wobei die Vorrichtung eingerichtet ist zur Bestimmung der definierten Menge von Kohlenmonoxid anhand des Volumens des Kohlenmonoxidreservoirs (15) und der über die Sensoren (17, 19) bestimmten Temperatur und Druck des Kohlenmonoxids im Kohlenmonoxidreservoir (15).

2. Vorrichtung gemäß einem der vorhergehenden Ansprüche, wobei ein Kohlendioxidfilter (51) in dem geschlossenen Gas-Volumen (100) angeordnet ist, der eingerichtet ist Kohlendioxid aus dem geschlossenen Gas-Volumen (100) herauszufiltern.

3. Vorrichtung gemäß einem der vorhergehenden Ansprüche, wobei die Vorrichtung ein Mittel zur Sauerstoffzufuhr aufweist, wobei das Mittel eingerichtet ist Sauerstoff dem geschlossenen Gas-Volumen (100) zuzuführen.

4. Vorrichtung gemäß dem vorhergehenden Anspruch, wobei das Mittel zur Sauerstoffzufuhr einen elastischen Ballon (41) umfasst, wobei der elastische Ballon (41) im Betrieb ein maximales Füllvolumen von 6 Liter, vorzugsweise 4,5 Liter und besonders bevorzugt 3 Liter aufweist.

5. Vorrichtung gemäß dem vorhergehenden Anspruch, aufweisend ein Mittel zur Bestimmung des Füllvolumens (55) des elastischen Ballons (41), wobei das Mittel (55) vorzugsweise ein optisches Mittel ist, das eingerichtet ist einen Abstand zwischen dem optischen Mittel und der Hülle des elastischen Ballons (41) zu messen, wobei das optische Mittel vorzugsweise zumindest einen Laser und einen Detektor umfasst, wobei der Laser und der Detektor unbeweglich an der Vorrichtung angeordnet sind.

6. Vorrichtung gemäß einem der vorhergehenden Ansprüche, wobei die Vorrichtung einen Kohlenmonoxiddetektor (53) aufweist, der eingerichtet ist den Kohlenmonoxidgehalt des geschlossenen Gas-Volumens (100) zu detektieren, wobei die Vorrichtung vorzugsweise einen weiteren Gasauslass (49) aufweist, wobei der Kohlenmonoxiddetektor an dem weiteren Gasauslass (49) angeordnet ist.

7. Vorrichtung gemäß dem vorhergehenden Anspruch, wobei das Mittel zur Kohlenmonoxidzufuhr und/oder das Mittel zur Sauerstoffzufuhr und/oder die Gasauslässe über Ventile (5, 13, 23, 25, 35, 37, 43, 45) mit dem geschlossenen Gas-Volumen verbunden ist, wobei die Ventile (5, 13, 23, 25, 35, 37, 43, 45) separat regelbar sind.

8. Vorrichtung gemäß einem der vorhergehenden Ansprüche, wobei der zumindest eine Drucksensor (17) des Kohlenmonoxidreservoirs (15) eine Messungenauigkeit von <100 mbar, vorzugsweise <50 mbar und besonders bevorzugt <20 mbar aufweist.

9. Vorrichtung gemäß einem der vorhergehenden Ansprüche, wobei der zumindest eine Temperatursensor (19) des Kohlenmonoxidreservoirs (15) eine Messungenauigkeit von <2 °C, vorzugsweise <1,2 °C und besonders bevorzugt <0,6 °C aufweist.

10. Vorrichtung gemäß einem der vorhergehenden Ansprüche, wobei die Vorrichtung eine Steuereinrichtung aufweist, wobei die Steuereinrichtung das Volumen des Kohlenmonoxids im Kohlenmonoxidreservoir (15) bestimmt mit einer Messungenauigkeit von <10 ml, vorzugsweise <5 ml und besonders bevorzugt <2,4 ml.

11. Vorrichtung gemäß einem der Ansprüche 3-10, wobei das Mittel zur Kohlenmonoxidzufuhr und/oder das Mittel zur Sauerstoffzufuhr zumindest einen regelbaren Anschluss aufweist zum Zuführen und/oder Abführen von Kohlenmonoxid bzw. Sauerstoff.

12. Vorrichtung gemäß einem der vorhergehenden Ansprüche, wobei alle Bauteile der Vorrichtung außer dem Mundstück (47) und dem Kohlendioxidfilter (51) in einem Gehäuse angeordnet sind.

13. Verfahren zur Bestimmung einer Hämoglobinmenge unter Verwendung einer Vorrichtung gemäß einem der vorhergehenden Ansprüche, das Verfahren aufweisend die Schritte:
i) Zuführen einer definierten Menge von Kohlenmonoxid zu dem geschlossenen Gas-Volumen (100);
ii) Ein- und Ausatmen des Gases im geschlossenen Gas-Volumen (100) durch den Patienten;
iii) Nicht-invasives Bestimmen der Menge von Kohlenmonoxid im Blut des Patienten;
iv) Bestimmen des Hämoglobin-Wertes anhand der Menge von Kohlenmonoxid im Blut des Patienten.

14. Verfahren gemäß einem der vorhergehenden Ansprüche 12-13, wobei das Verfahren vor dem Schritt i) die Schritte aufweist:
Zuführen von reinem Sauerstoff in das geschlossene Gas-Volumen (100); und
Ein- und Ausatmen des zugeführten reinen Sauerstoffs durch den Patienten.

15. Verfahren gemäß dem vorhergehenden Anspruch, wobei das Ein- und Ausatmen des Sauerstoffs vor dem Schritt i) für eine erste Zeit erfolgt und das Ein- und Ausatmen des Gases in Schritt ii) für eine zweite Zeit erfolgt, wobei die erste Zeit 0,1-6 Minuten beträgt, vorzugsweise 1-4 Minuten, und wobei die zweite Zeit 1-240 Minuten beträgt, vorzugsweise 2-10 Minuten, besonders bevorzugt 6-10 Minuten.

## Claims

1. A device for administering carbon monoxide for a determination of the hemoglobin amount of a patient comprising:
a closed gas volume (100);
a gas outlet comprising a mouth piece (47), wherein the mouth piece (47) is configured to enable inhalation and exhalation of gas into and out of the closed gas volume (100);
a means for carbon monoxide supply into the closed gas volume (100);
wherein the means for carbon monoxide supply comprises a carbon monoxide reservoir (15) with a volume which is provided with sensors for the determination of temperature (19) and pressure (17) of the carbon monoxide in the carbon monoxide reservoir (15),
wherein the means for carbon monoxide supply is configured to supply a defined amount of carbon monoxide into the closed gas volume (100),
wherein the device is adapted to determine the defined amount of carbon monoxide based on the volume of the carbon monoxide reservoir (15) and the temperature and pressure of the carbon monoxide in the carbon monoxide reservoir (15) determined by the sensors (17, 19).

2. Device according to one of the preceding claims, wherein a carbon dioxide filter (51) is arranged in the closed gas volume (100), which is configured to filter out carbon dioxide from the closed gas volume (100).

3. Device according to one of the preceding claims, wherein the device comprises a means for oxygen supply, wherein the means is configured to supply oxygen to the closed gas volume (100).

4. Device according to the preceding claim, wherein the means for oxygen supply comprises an elastic balloon (41), wherein the elastic balloon (41) comprises during operation a maximum filling volume of 6 liters, preferably 4.5 liters and particularly preferred 3 liters.

5. Device according to the preceding claim, comprising a means for determination of the filling volume (55) of the elastic balloon (41), wherein the means (55) is preferably an optical means, which is configured to detect the distance between the optical means and the envelope of the elastic balloon (41), wherein the optical means preferably comprises at least a laser and a detector, wherein the laser and the detector are fixedly arranged at the device.

6. Device according to one of the preceding claims, wherein the device comprises a carbon monoxide detector (53), which is configured to detect the carbon monoxide content of the closed gas volume (100), wherein the device preferably comprises a further gas outlet (49), wherein the carbon monoxide detector is arranged at said further gas outlet (49).

7. Device according to the preceding claim, wherein the means for carbon monoxide supply and/or the means for oxygen supply and/or the gas outlets are connected to the closed volume via valves (5, 13, 23, 25, 35, 37, 43, 45); wherein the valves (5, 13, 23, 25, 35, 35, 37, 43, 45) are separately adjustable.

8. Device according to one of the preceding claims, wherein the at least one pressure sensor (17) of the carbon monoxide reservoir (15) comprises a measurement inaccuracy of <100 mbar, preferably <50mbar and particularly preferred <20 mbar.

9. Device according to one of the preceding claims, wherein the at least one temperature sensor (19) of the carbon monoxide reservoir (15) comprises a measurement inaccuracy of <2°C, preferably <1.2°C and particularly preferred <0.6°C.

10. Device according to one of the preceding claims, wherein the device comprises a control device, wherein the control device determines the volume of the carbon monoxide in the carbon monoxide reservoir (15) with a measurement inaccuracy of <10 ml, preferably <5 ml and particularly preferred <2.4 ml.

11. Device according to one of the claims 3-10, wherein the means for carbon monoxide supply and/or the means for oxygen supply comprise at least one adjustable part for supplying or discharging carbon monoxide or oxygen.

12. Device according to one of the preceding claims, wherein all parts of the device, except the mouth piece (47) and the carbon dioxide filter (51) are arranged in a housing.

13. Method for the determination of a hemoglobin amount using a device according to one of the preceding claims, wherein the method comprises the steps:
i) supplying a defined amount of carbon monoxide to the closed gas volume (100);
ii) in- and exhaling the gas in the closed gas volume (100) by the patient;
iii) non-invasive determination of the amount of carbon monoxide in the blood of the patient;
iv) determination of the hemoglobin value based on the amount of carbon monoxide in the blood of the patient.

14. Method according to one of the preceding claims 12-13, wherein the method comprises before step i) the steps:
supplying pure oxygen into the closed gas volume (100); and
in- and exhaling of the supplied pure oxygen by the patient.

15. Method according to the preceding claim, wherein the in- and exhaling of the oxygen before the step i) occurs for a first duration and the in- and exhaling of the gas in step ii) occurs for a second duration, wherein the first duration is 0.1-6 minutes, preferably 1-4 minutes, and wherein the second duration is 1-240 minutes, preferably 2-10 minutes, particularly preferred 6-10 minutes.

## Revendications

1. Un dispositif destiné à administrer du monoxyde de carbone pour déterminer la quantité d'hémoglobine d'un patient, comprenant :
un volume de gaz fermé (100) ;
une sortie de gaz comportant un embout (47), l'embout (47) étant conçu pour permettre une inspiration et une expiration de gaz dans et à partir du volume de gaz fermé (100) ;
un moyen d'amenée de monoxyde de carbone dans le volume de gaz fermé (100) ;
le moyen d'amenée de monoxyde de carbone comprenant un réservoir (15) de monoxyde de carbone ayant un volume qui est pourvu de capteurs destinés à déterminer la température (19) et la pression (17) du monoxyde de carbone dans le réservoir (15) de monoxyde de carbone, le moyen d'amenée de monoxyde de carbone étant conçu pour amener au volume de gaz fermé (100) une quantité définie de monoxyde de carbone,
dans lequel le dispositif est conçu pour déterminer la quantité définie de monoxyde de carbone à l'aide du volume du réservoir (15) de monoxyde de carbone et de la température et de la pression du monoxyde de carbone, déterminées grâce aux capteurs (17, 19), dans le réservoir (15) de monoxyde de carbone.

2. Dispositif selon l'une des revendications précédentes, dans lequel un filtre (51) de dioxyde de carbone est disposé dans le volume de gaz fermé (100), qui est conçu pour séparer par filtration le dioxyde de carbone du volume de gaz fermé (100).

3. Dispositif selon l'une des revendications précédentes, dans lequel le dispositif comprend un moyen d'amenée d'oxygène, le moyen étant conçu pour amener de l'oxygène au volume de gaz fermé (100).

4. Dispositif selon la revendication précédente, dans lequel le moyen d'amenée d'oxygène comprend un ballon élastique (41), le ballon élastique (41) présentant en cours d'utilisation un volume de remplissage maximal de 6 litres, de préférence de 4,5 litres et d'une manière particulièrement préférée de 3 litres.

5. Dispositif selon la revendication précédente, comprenant un moyen permettant de déterminer le volume de remplissage (55) du ballon élastique (41), dans lequel le moyen (55) est de préférence un moyen optique qui est conçu pour mesurer une distance entre le moyen optique et l'enveloppe du ballon élastique (41), le moyen optique comprenant de préférence au moins un laser et un détecteur, le laser et le détecteur étant disposés d'une manière fixe contre le dispositif.

6. Dispositif selon l'une des revendications précédentes, dans lequel le dispositif comprend un détecteur (53) de monoxyde de carbone qui est conçu pour détecter la teneur en monoxyde de carbone du volume de gaz fermé (100), le dispositif comprenant de préférence une sortie de gaz supplémentaire (49), le détecteur de monoxyde de carbone étant disposé contre la sortie de gaz supplémentaire (49).

7. Dispositif selon la revendication précédente, dans lequel le moyen d'amenée de monoxyde de carbone et/ou le moyen d'amenée d'oxygène et/ou les sorties de gaz sont reliés par l'intermédiaire de vannes (5, 13, 23, 25, 35, 37, 43, 45) au volume de gaz fermé, les vannes (5, 13, 23, 25, 35, 37, 43, 45) pouvant être réglées séparément.

8. Dispositif selon l'une des revendications précédentes, dans lequel l'au moins un capteur de pression (17) du réservoir (15) de monoxyde de carbone présente une précision de mesure < 100 mbar, de préférence < 50 mbar et d'une manière particulièrement préférée < 20 mbar.

9. Dispositif selon l'une des revendications précédentes, dans lequel l'au moins un capteur de température (19) du réservoir (15) de monoxyde de carbone présente une précision de mesure < 2 °C, de préférence < 1,2 °C et d'une manière particulièrement préférée < 0,6 °C.

10. Dispositif selon l'une des revendications précédentes, dans lequel le dispositif comprend un dispositif de commande, le dispositif de commande déterminant le volume du monoxyde de carbone dans le réservoir (15) de monoxyde de carbone avec une précision de mesure < 10 ml, de préférence < 5 ml et d'une manière particulièrement préférée < 2,4 ml.

11. Dispositif selon l'une des revendications 3 à 10, dans lequel le moyen d'amenée de monoxyde de carbone et/ou le moyen d'amenée d'oxygène comprennent au moins un raccord réglable pour amener et/ou évacuer respectivement le monoxyde de carbone et l'oxygène.

12. Dispositif selon l'une des revendications précédentes, dans lequel tous les composants du dispositif, à part l'embout (47) et le filtre (51) de dioxyde de carbone, sont disposés dans un boîtier.

13. Procédé de détermination d'une quantité d'hémoglobine par utilisation d'un dispositif selon l'une des revendications précédentes, le procédé comprenant les étapes suivantes :
i) amenée d'une quantité définie de monoxyde de carbone au volume de gaz fermé (100) ;
ii) inspiration et expiration, par le patient, du gaz se trouvant dans le volume de gaz fermé (100) ;
iii) détermination non invasive de la quantité de monoxyde de carbone dans le sang du patient ;
iv) détermination de la valeur globulaire à l'aide de la quantité de monoxyde de carbone dans le sang du patient.

14. Procédé selon la revendication 13 précédente, dans lequel le procédé comprend avant l'étape i) les étapes suivantes :
amenée d'oxygène pur dans le volume de gaz fermé (100) ; et
inspiration et expiration, par le patient, de l'oxygène pur amené.

15. Procédé selon la revendication précédente, dans lequel l'inspiration et l'expiration de l'oxygène avant l'étape i) s'effectuent pendant un premier temps, et l'inspiration et l'expiration du gaz dans l'étape ii) s'effectuent pendant un second temps, le premier temps étant de 0,1 à 6 minutes, de préférence de 1 à 4 minutes, et le second temps étant de 1 à 240 minutes, de préférence de 2 à 10 minutes, d'une manière particulièrement préférée de 6 à 10 minutes.
